# EUROPÄISCHE PATENTSCHRIFT

(11) **EP 0 502 374 B1**
(45) Veröffentlichungstag und Bekanntmachung des Hinweises auf die Patenterteilung: **05.07.1995**
(21) Anmeldenummer: 92102911.2
(22) Anmeldetag: 21.02.1992
(51) Int. Cl.: C07C 45/38, C07C 67/313, C07D 319/06

(54) **Verfahren zur Herstellung von 3-Alkoxycarbonylpropenalen und 3-Dialkoxymethylpropenalen**
Process of preparation of 3-alkoxycarbonylpropenals and 3-dialkoxymethylpropenals
Procédé pour la préparation des 3-alkoxycarbonylpropénals et 3-dialkoxyméthylpropénals

(30) Priorität: 05.03.1991 DE 4106907
(43) Veröffentlichungstag der Anmeldung: 09.09.1992
(73) Patentinhaber: BASF Aktiengesellschaft, 67063 Ludwigshafen (DE)
(72) Erfinder: Aquila, Werner, Dr., W-6800 Mannheim 31 (DE); Scholz, Hans-Ulrich, Dr., W-6719 Weisenheim (DE); Fuchs, Hartwig, W-6700 Ludwigshafen (DE); Krause, Wolfgang, Dr., W-6800 Mannheim 81 (DE); Paust, Joachim, Dr., W-6708 Neuhofen (DE); Hoffmann, Werner, Dr., W-6708 Neuhofen (DE)

(56) Entgegenhaltungen:
- EP-A- 0 218 124
- EP-A- 0 231 920
- EP-A- 0 263 385
- EP-A- 0 287 884
- DE-A- 2 225 612
- DE-A- 3 603 662
- CANADIAN JOURNAL OF CHEMISTRY Bd. 57, Nr. 24, 15. Dezember 1979, CANADA Seiten 3354 - 3356; MASATOSHI KAKUSHIMA ET AL.: 'Total synthesis of (+)-5beta,8alpha-androst-9(11)-ene-3,17-dione'

## Beschreibung

Die vorliegende Erfindung betrifft ein neues Verfahren zur Herstellung von 3-Alkoxycarbonylpropenalen und 3-Dialkoxymethylpropenalen der allgemeinen Formeln Ia bzw. Ib 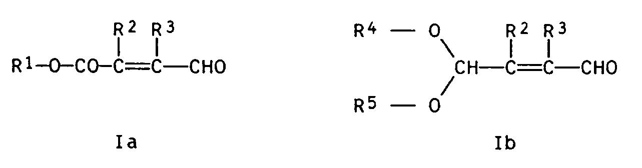
wobei R¹ eine C₁-C₃-Alkylgruppe bezeichnet, R² und R³ für Wasserstoff, Methyl oder Ethyl stehen und R⁴ und R⁵ C₁-C₄-Alkylgruppen bedeuten, die zu einem 5- oder 6-gliedrigen Ring verbunden sein können.

Die Verbindungen Ia und Ib sind bekannt und dienen als Zwischenprodukte für die Synthese von Carotinoiden.

In der US-A 3 118 930 ist die Herstellung der 3-Alkoxycarbonyl-propenale Ia, in denen der Rest R² Wasserstoff und R³ eine Methylgruppe bedeutet, durch Umsetzung eines 2-Methyl-3-brom-prop-1-en-carbon-säurealkylesters mit Pyridin zum Pyridiniumsalz und dessen Behandlung mit einer alkoholischen Lösung von p-Nitrosodialkylanilin in Gegenwart von Alkalihydroxid sowie anschließende Hydrolyse mit einer Säure beschrieben.

Aus Ars. Pharm., 29 (2), 117 (1988) ist die Umsetzung des 2-Methyl-3-brom-prop-1-en-carbonsäuremethylesters mit Dimethylsulfoxid und Natriumhydrogencarbonat zum entsprechenden 3-Methoxycarbonylpropenal Ia bekannt.

In Can. J. Chem. 57, 3354 (1979) ist die Reaktion eines Alkohols IIa, in dem R¹ eine Ethylgruppe und R² eine Methylgruppe bezeichnen und R³ für Wasserstoff steht, mit Mangandioxid zu dem entsprechenden 3-Ethoxy-carbonylpropenal Ia beschrieben.

Aus der DE-A 22 25 612 ist die Herstellung der 3-Dialkoxymethyl-propenale Ib, in denen einer der Reste R² oder R³ Wasserstoff und der andere eine Methylgruppe bezeichnet, durch Oxidation der entsprechenden Alkohole IIb in der Flüssigphase mit Pyridin-Schwefeltrioxid-Komplexen, Pyridin-Chromtrioxid-Komplexen, Managandioxid oder Nickelperoxid sowie mit schwerelasaurer Chromsäurelösung in Aceton bekannt.

Nach einem anderen Syntheseprinzip läßt sich ein Teil der Verbindungen IIb, in denen R² nur Wasserstoff bezeichnet, durch Aldolkondensation von Glyoxalmonoacetalen mit Aldehyden herstellen (DE-A 36 17 409).

Diese bisher bekannten Verfahren vermögen jedoch aufgrund des hohen technischen Aufwandes sowie des Verbrauchs teurer Oxidationsmittel nicht zu befriedigen.

Ferner ist aus der DE-A 36 03 662 die mit Metallen der Gruppe IB des Periodensystems katalysierte Gasphasen-Dehydrierung von gesättigten monoacetalisierten Alkoholen mit Sauerstoff oder einem Sauerstoff enthaltenen Gas zu gesättigten monoacetalisierten Aldehyden bekannt.

Weiterhin ist aus EP-A-263 385 die mit speziellen Trägerkatalysatoren von Kupfer, Silber und/oder Gold katalysierte Gasphasendehydrierung von gesättigten oder ungesättigten Alkoholen der Formel R-CH₂OH zu den Aldehyden der Formel R-CHO bekannt.

Der Erfindung lag die Aufgabe zugrunde, 3-Alkoxycarbonylpropenale Ia und 3-Dialkoxymethylpropenale Ib auf einfachere und wirtschaftlichere Weise zugänglich zu machen.

Es wurde nun überraschenderweise gefunden, daß auch die als außerordentlich reaktionsfreudig bekannten 3-Alkoxycarbonyl- und 3-Dialkoxymethyl-propenale der allgemeinen Formeln Ia bzw. Ib durch die mit Metallen der Gruppe IB des Periodensystems katalysierte Gasphasen-Dehydrierung der entsprechenden Alkohole mit überraschend hohen Selektivitäten erhalten werden können.

Gegenstand der Erfindung ist ein Verfahren zur Herstellung von 3-Alkoxycarbonylpropenalen und 3-Dialkoxymethylpropenalen der allgemeinen Formeln Ia bzw. Ib 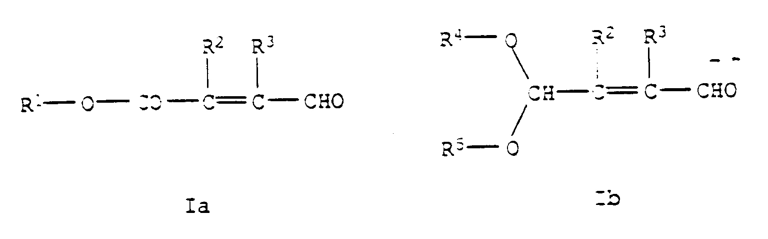
wobei R¹ eine C₁-C₃-Alkylgruppe bezeichnet, R² und R³ für Wasserstoff, Methyl oder Ethyl stehen und R⁴ und R⁵ C₁-C₄-Alkylgruppen bedeuten, die zu einem 5- oder 6-gliedrigen Ring verbunden sein können, welches dadurch gekennzeichnet ist, daß man einen entsprechenden Alkohol der allgemeinen Formel IIa oder IIb 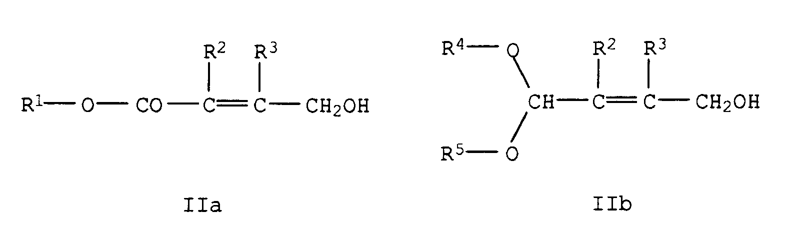
mit Sauerstoff oder einem sauerstoffhaltigen Gas in der Gasphase in Gegenwart eines Metalls der Gruppe IB des Periodensystems oder einer Verbindung eines dieser Metalle als Katalysatoren umsetzt.

Die Ausgangsverbindungen II sind bekannt oder nach bekannten Methoden erhältlich. Die Verbindungen IIa werden beispielsweise durch Umsetzung von 1-Methyl-prop-2-on-carbonsäureethylester mit m-Chlorperbenzoesäure zum entsprechenden Epoxid und anschließender Behandlung dieser Epoxide mit Kaliumcarbonat in Ethanol hergestellt (Can. J. Chem., 57, 3354 (1979)). Die Ausgangsverbindungen IIb sind z.B. durch Acetalisierung von 4-Acetoxy-but-2-en-1-alen mit Alkoholen und anschließende Überführung dieser Verbindungen durch Abspaltung der Acetylgruppen in die freien Alkohole erhältlich (Liebigs Ann. Chem., 2194 (1976)).

Im Hinblick auf die gewünschten Verfahrensprodukte I sind die Verbindungen II besonders bevorzugt, in denen einer der Reste R² und R³ Wasserstoff und der andere eine Methylgruppe bedeutet.

Aus verfahrenstechnischen Gründen haben diejenigen Verbindungen II die größte Bedeutung, in denen die Reste R⁴ und R⁵ gemeinsam für den Neopentylenrest stehen.
Weiterhin bevorzugt sind solche Reste R⁴ und R⁵, die sich vom 1,2-Ethylenglykol oder 1,3-Propylenglykol ableiten und offenkettige Reste wie vor allem Methyl.

Die im erfindungsgemäßen Verfahren einzusetzenden Katalysatoren sind Metalle der Gruppe IB des Periodensystems oder Verbindungen eines dieser Metalle.

Man kann die Katalysatoren in metallischer Form, in Form von Salzen und Oxiden einsetzen.

Als Katalysatoren in metallischer Form eignen sich bevorzugt Kupfer und insbesondere Silber. Vorzugsweise verwendet man die Metalle in Form von Trägerkatalysatoren. Bevorzugte Träger sind anorganische Materialien wie keramische Massen, z.B. Porzellan, sowie Magnesiumoxid, Aluminiumoxid, Siliciumoxid, Siliciumcarbid, Titanoxid, Zinkoxid, Lanthanoxid, Aluminiumsilikat oder Gemische dieser Materialien sowie vor allem Steatit, wobei Silber auf Steatit besonders hervorzuheben ist.

Die Trägerkatalysatoren haben vorzugsweise einen Gehalt an aktiven Metallen der Gruppe IB von 0,01 bis 50, besonders 0,05 bis 30 und ganz besonders von 1 bis 20 Gew.-%, bezogen auf die Summe aus Träger und katalytisch aktiven Metallen, berechnet als Metall.

Die Katalysatoren können wahlweise als 2- bis 4-mm-Stränge oder als Tabletten mit 3 bis 5 mm Durchmesser, als Splitt mit Teilchengrößen von 0,05 bis 1 mm, vorzugsweise 0,1 bis 0,5 mm als größten Durchmesser oder als Pulver mit Teilchengrößen von 0,05 bis 0,5 mm eingesetzt werden, wobei sich das Pulver auch als Wirbelkontakt eignet. Besonders bevorzugt sind Schalenkatalysatoren in Kugelform mit einem Durchmesser von 1 bis 10 mm mit Steatit als Kernmaterial.

Die Katalysatorbelastung liegt beim kontinuierlichen Verfahren im allgemeinen zwischen 0,2 und 2 t, insbesondere 0,3 und 1,5 t der Verbindung II pro m² Katalysatorquerschnitt und Stunde.

Die Oxidation der Ausgangsstoffe II zu den Verfahrensprodukten I erfolgt durch Sauerstoff oder ein sauerstoffhaltiges Gas, wobei Luft als oxidierendes Agens besonders bevorzugt ist.

Das Molverhältnis von Sauerstoff zu den Ausgangsverbindungen II beträgt zweckmäßigerweise 0,1:1 bis 4:1, besonders 0,2:1 bis 2:1.

In der Regel führt man die Reaktion bei 300 bis 700°C, vorzugsweise bei 320 bis 500°C durch.

Im allgemeinen wird die Umsetzung bei Drücken von 0,0005 bis 2 bar, insbesondere von 0,001 bis 1 bar vorgenommen. Die Verweilzeiten des Gasgemisches im Reaktionsraum betragen normalerweise 0,0005 bis 1 Sekunden, meistens 0,001 bis 0,5 Sekunden.

Es empfiehlt sich, Sauerstoff nicht in reiner Form, sondern in Mischung mit einem Inertgas im Volumenverhältnis des Inertgases zu Sauerstoff von bevorzugt 1:1 bis 50:1, besonders 3:1 bis 20:1 einzusetzen. Bevorzugt sind Kohlenoxid, Kohlendioxid, Edelgase und vor allem Stickstoff. Besonders bevorzugt wird Luft als oxidierendes Gas verwendet.

Um die Ausgangsverbindungen II leichter in die Gasphase zu überführen, empfiehlt es sich, sie in Form ihrer Lösungen einzusetzen und gemeinsam mit dem Lösungsmittel zu verdampfen. Bevorzugte Lösungsmittel sind Wasser und vor allem organische Lösungsmittel. Als organische Lösungsmittel eignen sich besonders Ketone wie Aceton und Methylethylketon, Ether wie Tetrahydrofuran, Dioxan und Diisopropylether, Ester wie Essigsäureethylester sowie Kohlenwasserstoffe wie Toluol. In der Regel verwendet man das 0,05- bis 5-fache, vorzugsweise das 0,1- bis 3-fache der Ausgangsverbindung II. Um den Verdampfungsvorgang zu erleichtern, kann man auch ein Trägergas wie eines der vorgenannten Inertgase, bevorzugt Stickstoff, mitverwenden.

Die Oxidation erfolgt in der Gasphase, wobei eine Festbettreaktion und eine Gasphasenreaktion im Wirbelbett in Betracht kommen. Eine wirtschaftlich sehr vorteilhafte Ausführungsform des erfindungsgemäßen Verfahrens besteht in der Durchführung an einem festen Katalysatorbett, indem man den Alkohol II, gegebenenfalls zusammen mit einem Lösungsmittel und/oder einem Trägergas verdampft und als Gasphase, bevorzugt im Gemisch mit einem Inertgas, mit Sauerstoff oder einem sauerstoffhaltigen Gas über einen festen Katalysator leitet.

Die Aufarbeitung des Reaktionsgemisches auf die Verfahrensprodukte I erfolgt in an sich bekannter Weise, und zwar in der Regel durch Abkühlung der Reaktionsgase und Absorption in einem inerten Lösungsmittel. Anschließend kann man das Verfahrensprodukt noch durch fraktionierende Destillation reinigen. Als inerte Lösungsmittel eignen sich bevorzugt Hexan, Toluol, Methyl-tert.-butylether und vor allem Wasser sowie insbesondere das Verfahrensprodukt selber.

Es empfiehlt sich, insbesondere zur Unterdrückung der säurekatalysierten Bisacetalbildung, die Reaktionsausträge mit Basen wie Alkali- und Erd-alkalihydroxiden, Alkali- und Erdalkalicarbonaten bzw. -hydrogencarbonaten zu stabilisieren. Bevorzugt sind Natriumcarbonat und -hydrogencarbonat. Besonders gute Ergebnisse erzielt man mit wäßrigen Urotropinlösungen, deren Menge sich nach der Säurezahl der Reaktionsausträge richtet.

Die nach dem erfindungsgemäßen Verfahren auf einfache und wirtschaftliche Weise erhältlichen 3-Alkoxycarbonylpropenale Ia und 3-Dialkoxymethyl-propenale Ib dienen als Zwischenprodukte für die Synthese von Carotinoiden. So kann man beispielsweise durch Umsetzung von 3-Methyl-2-buten-1,4-dialdehyd-mono-1-acetalen Ib mit dem γlid von β-Jonylidenethyltriphenylphosphoniumsalzen und anschließende Hydrolyse in Gegenwart wäßriger Säuren Retinal (Vitamin-A₁-Aldehyd) herstellen. Im Falle der entsprechenden Verbindung Ia gelangt man zu Retinsäure estern bzw. Retinsäure. Das Retinal kann durch einfache Wittig-Olefinierung mit dem Retinyltriphenylphosphoniumsalz sowie nachfolgende Hydrolyse in β-Carotin überführt werden. Bei Umsetzung der Retinyltriphenylphosphoniumsalze mit 2-Methyl-2-buten-1,4-dialdehyd-mono-1-acetalen Ib in einer Wittig-Reaktion und anschließender Hydrolyse erhält man das β-Apo-C₂₅-Carotinal.

### Beispiele

1. Herstellung von 2-Methyl-but-2-en-1,4-dial-1-neopentylglykol-acetal
   Innerhalb einer Stunde wurden 50 g 4-Hydroxy-2-methyl-but-2-en-1-neo-pentylglykol-acetal mit 12,8 l Luft bei einem Druck von 80 mbar und einer Temperatur von 435°C verdampft und innerhalb von 0,2 Sekunden über 12 g eines festen Silberkatalysators (4 Gew.-% Silber auf Steatit) geleitet.
   Die übliche Aufarbeitung durch Absorption des Reaktionsaustrags mit 10%iger Urotropinlösung lieferte das 2-Methyl-but-2-en-1,4-dial-1-neopentylglykol-acetal in einer Ausbeute von 63 % und einer Selektivität von 90 %.
2. Herstellung von 3-Methyl-but-2-en-1,4-dial-1-neopentylglykol-acetal
   Analog Beispiel 1 wurden pro Stunde 50 g 4-Hydroxy-3-methyl-but-2-en-1-neopentylglykol-acetal mit 10,4 l Luft bei einem Druck von 80 mbar und einer Temperatur von 430°C über den Silberkatalysator geleitet und der Rohaustrag wurde anschließend mit 10%iger Urotropinlösung behandelt.
   Man erhielt das 3-Methyl-but-2-en-1,4-dial-1-neopentylglykol-acetal in einer Ausbeute von 83 % und einer Selektivität von 93 %.
3. Herstellung von 2-Methyl-4-oxo-2-buten-säureethylester
   Analog Beispiel 1 wurden pro Stunde 60 g 4-Hydroxy-2-methyl-2-butensäureethylester mit 12,8 l Luft bei einem Druck von 500 mbar und einer Temperatur von 400°C über den Silberkatalysator geleitet und der Rohaustrag wurde anschließend fraktionierend destilliert.
   Man erhielt den 2-Methyl-4-oxo-2-buten-säureethylester in einer Ausbeute von 68 % und einer Selektivität von 75 %.

## Patentansprüche

1. Verfahren zur Herstellung von 3-Alkoxycarbonylpropenalen und 3-Dialkoxymethylpropenalen der allgemeinen Formel Ia bzw. Ib 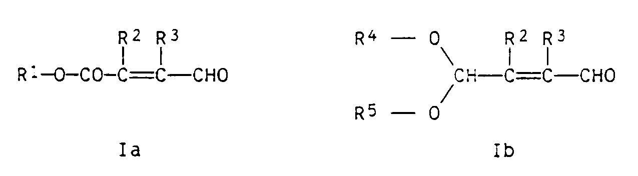 wobei R¹ eine C₁-C₃-Alkylgruppe bezeichnet, R² und R³ für Wasserstoff, Methyl oder Ethyl stehen und R⁴ und R⁵ C₁-C₄-Alkylgruppen bedeuten, die zu einem 5- oder 6-gliedrigen Ring verbunden sein können, dadurch gekennzeichnet, daß man einen entsprechenden Alkohol der allgemeinen Formel IIa oder IIb 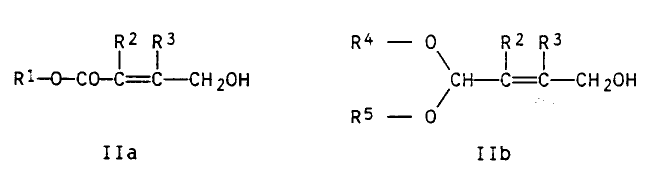 mit Sauerstoff oder einem sauerstoffhaltigen Gas in der Gasphase in Gegenwart eines Metalls der Gruppe IB des Periodensystems oder einer Verbindung eines dieser Metalle als Katalysatoren umsetzt.

2. Verfahren nach Anspruch 1, dadurch gekennzeichnet, daß R⁴ und R⁵ gemeinsam für den Neopentylenrest 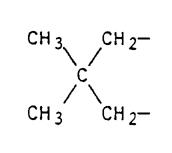 stehen.

3. Verfahren nach den Ansprüchen 1 oder 2, dadurch gekennzeichnet, daß man die Reaktion in Gegenwart von Silberkatalysatoren als Katalysatoren vornimmt.

4. Verfahren nach Anspruch 1, dadurch gekennzeichnet, daß man den Alkohol der Formel IIa oder IIb bei Drücken von 0,001 bis 1 bar mit Sauerstoff oder einem sauerstoffhaltigen Gas umsetzt.

5. Verfahren gemäß Anspruch 1, dadurch gekennzeichnet, daß man die Reaktionsausträge zur Unterdrückung der säurekatalysierten Bisacetalbildung mit Basen stabilisiert.

6. Verfahren nach Anspruch 5, dadurch gekennzeichnet, daß man die Reaktionsausträge mit einer wäßrigen Urotropinlösung stabilisiert.

## Claims

1. A process for preparing 3-alkoxycarbonylpropenals and 3-dialkoxymethylpropenals of the general formula Ia or Ib respectively 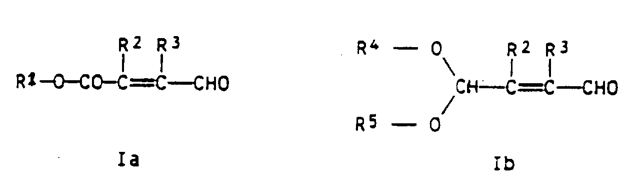 where R¹ is C₁-C₃-alkyl, R² and R³ are hydrogen, methyl or ethyl, and R⁴ and R⁵ are C₁-C₄-alkyl groups which can be connected to form a 5- or 6-membered ring, which comprises reacting a corresponding alcohol of the general formula IIa or IIb 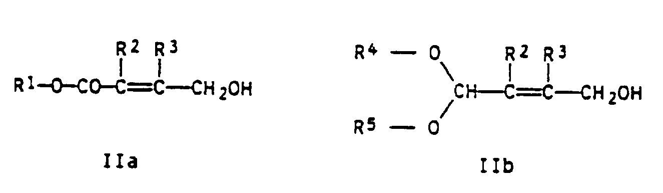 with oxygen or an oxygen-containing gas in the gas phase in the presence of a metal of group IB of the periodic table or of a compound of one of these metals as catalysts.

2. A process as claimed in claim 1, wherein R⁴ and R⁵ together are the neopentyl radical

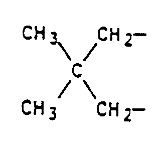

3. A process as claimed in claim 1 or 2, wherein the reaction is carried out in the presence of silver catalysts.

4. A process as claimed in claim 1, wherein the alcohol of the formula IIa or IIb is reacted under pressures of from 0.001 to 1 bar with oxygen or an oxygen-containing gas.

5. A process as claimed in claim 1, wherein the discharges from the reaction are stabilized with bases to suppress acid-catalyzed bisacetal formation.

6. A process as claimed in claim 5, wherein the discharges from the reaction are stabilized with an aqueous Urotropin solution.

## Revendications

1. Procédé de préparation de 3-alcoxycarbonylpropénal et 3-dialcoxyméthylpropénal de la formule générale Ia ou Ib 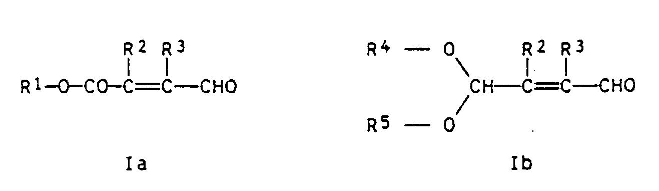 R¹ désignant un groupe alkyle en C1-C3, R² et R³ étant mis pour hydrogène, méthyle ou éthyle et R⁴ et R⁵ désignant des groupes alkyle en C1-C4 qui peuvent être liés en un noyau de 5 à 6 chaînons, caractérisé par le fait qu'on fait réagir un alcool correspondant de la formule générale IIa ou IIb 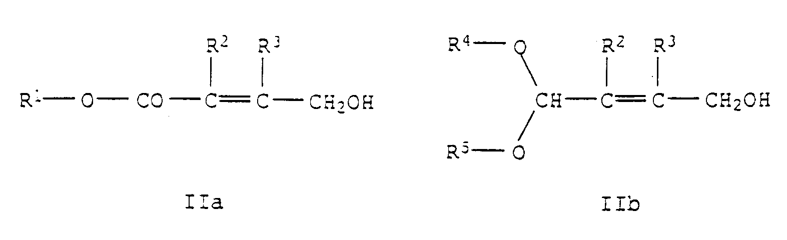 avec de l'oxygène ou un gaz oxygéné dans la phase gazeuse, en présence d'un métal du groupe Ib du système périodique ou d'un composé d'un de ces métaux comme catalyseurs.

2. Procédé selon la revendication 1,caractérisé par le fait que R⁴ et R⁵ sont ensemble mis pour le reste néopentylène.

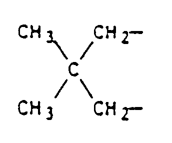

3. Procédé selon les revendications 1 ou 2, caractérisé par le fait que l'on effectue la réaction en présence de catalyseurs d'argent comme catalyseurs.

4. Procédé selon la revendication 1 caractérisé par le fait qu'on fait réagir l'alcool de formule IIa ou IIb, à des pressions de 0,001 à 1 bar, avec de l'oxygène ou un gaz oxygéné.

5. Procédé selon la revendication 1 caractérisé par le fait qu'on stabilise les produits de la réaction avec des bases pour affaiblir la formation de bisacétal catalysé par les acides.

6. Procédé selon la revendication 5, caractérisé par le fait qu'on stabilise les produits de la réaction avec une solution aqueuse d'urotropine.
